(19) Europäisches Patentamt
European Patent Office
Office européen des brevets

(11) Veröffentlichungsnummer: **0 121 752**
**A2**

(12) # EUROPÄISCHE PATENTANMELDUNG

(21) Anmeldenummer: **84102343.5**

(51) Int. Cl.³: **A 61 K 39/39**

(22) Anmeldetag: **05.03.84**

(30) Priorität: **10.03.83 DE 3308458**

(43) Veröffentlichungstag der Anmeldung: **17.10.84 Patentblatt 84/42**

(84) Benannte Vertragsstaaten: **AT BE CH DE FR GB IT LI LU NL SE**

(71) Anmelder: **BEHRINGWERKE Aktiengesellschaft, Postfach 1140, D-3550 Marburg 1 (DE)**

(72) Erfinder: **Bernhardt, Dieter, Dr., Goldbergstrasse 18a, D-3553 Cölbe (DE)**
Erfinder: **Mauler, Rudolf, Dr., Finkenstrasse 6, D-3550 Marburg 7 (DE)**
Erfinder: **Bengelsdorff, Hans-Joachim, Dr., Oberer Eichweg 39, D-3550 Marburg 1 (DE)**

(74) Vertreter: **Meyer-Dulheuer, Karl-Hermann, Dr. et al, HOECHST Aktiengesellschaft Zentrale Patentabteilung Postfach 80 03 20, D-6230 Frankfurt/Main 80 (DE)**

(54) **Verfahren zur Adjuvierung von Vaccinen.**

(57) Es wird ein Verfahren zur Adjuvierung von Vaccinen ohne Verwendung eines Öls beschrieben, worin die Adjuvantien organisch-chemische Verbindungen sind.

EP 0 121 752 A2

0121752

BEHRINGWERKE AKTIENGESELLSCHAFT HOE 83/B 003 Dr.HA

## Verfahren zur Adjuvierung von Vaccinen

Die Erfindung betrifft ein Verfahren zur Adjuvierung von Vaccinen ohne Verwendung eines Öls.

Viele Antigene sind sowenig immunogen, daß sie bei einer einmaligen Injektion in ein Tier keine meßbare oder nur geringe Immunantwort auslösen, so daß sie nicht ohne weiteres als Impfstoff verwendet werden können. Die antigene Wirksamkeit kann verstärkt werden, indem diese Antigene mit einem Immunstimulator oder Adjuvans kombiniert werden. Die meisten inaktivierten Virus- und Bakterienvaccinen enthalten aus diesem Grund Adjuvantien.

Obwohl eine Reihe von Adjuvantien aus unterschiedlichen Stoffklassen, wie mineralische oder bakterielle Adjuvantien und Öladjuvantien, bekannt sind, ist es wünschenswert, verträglichere und wirksamere Adjuvantien aufzufinden. Hierbei wird der Abbaubarkeit der Adjuvantien im Organismus heute eine besonders starke Bedeutung beigemessen. Dieses Postulat erfüllen die mineralischen und Öladjuvantien nur teilweise oder überhaupt nicht, d.h. das verwendete Adjuvans bleibt lange an der Injektionsstelle (Immunological Adjuvans, Report of the WHO scientific group No. 595, Genf 1976).

Es wurde nun überraschenderweise gefunden, daß Polyole, das heißt Kondensationsprodukte mehrwertiger Alkohole und besonders solche der Formel I

$$HO(CH_2CH_2O)_a(\underset{CH_3}{CH}CH_2O)_b(CH_2CH_2O)_cH \qquad (I)$$

mit einem Molekulargewicht zwischen 1.000 und 15.000,

wobei a und c statistisch gleich sind und das Verhältnis a zu b von 1:10 bis zu 1:16 reicht, sowie Fettsäureester von mehrwertigen Alkoholen, besonders Ester natürlicher Fettsäuren wie Ölsäure, Stearinsäure, Palmitinsäure oder Laurinsäure mit Sorbitan, Mannitan, Hydroxygruppen tragende Polyoxyalkylenen oder Glycerin, insbesondere Monoester von Sorbitan mit den genannten natürlichen Fettsäuren, immunstimulierende Eigenschaften haben.

Es ist bekannt, die genannten Stoffe als Emulgatoren in Ölvaccinen zu verwenden. Es war jedoch bisher nicht bekannt, daß diese Stoffe eine immunstimulierende Wirkung haben.

Gegenstand der Erfindung ist deshalb ein Verfahren zur Adjuvierung ölfreier Vaccinen, dadurch gekennzeichnet, daß der Vaccine eine der genannten Verbindungen zugesetzt wird.

Bevorzugt wird eine Verbindung der Formel I mit einem durchschnittlichen Molekulargewicht von 3.800, wobei das Molekulargewicht des Polypropylenmittelteils etwa 3.250 beträgt, sowie besonders eine solche Verbindung mit einem durchschnittlichen Molekulargewicht von 2.000, wobei das Molekulargewicht des Mittelteils 1.750 beträgt.

Bevorzugt ist weiterhin ein Ester einer Fettsäure mit 10 bis 20 Kohlenstoffatomen mit einem mehrwertigen Alkohol mit 3 bis 6 Kohlenstoffatomen.

Besonders bevorzugt sind die Ester der Ölsäure, Stearinsäure, Palmitinsäure oder Laurinsäure mit Sorbitan, Mannitan, Hydroxygruppen tragenden Polyoxyalkylenen oder Glycerin, insbesondere Monoester.

Diese Verbindungen werden der Vaccine in einer Menge von 0,1 bis 30, vorzugsweise 1 bis 20 g auf 100 ml Vaccine zugesetzt.

Die Vaccinen können weiterhin übliche Hilfs- und Zusatzstoffe enthalten.

Die folgenden Beispiele sollen die Erfindung erläutern.

Beispiel 1

10 ml eines Gemischs, das inaktiviertes Reo 1-, PI 3- und IBR-Virusantigen enthielt, wurden in 2 x 5 ml aufgeteilt. 5 ml des Antigengemisches blieben ohne Adjuvans. Die anderen 5 ml wurden mit Pluronic® L 61 (Verbindung der Formel I, wobei das Molekulargewicht dieser Verbindung 2.000 und das des Mittelteils 1.750 ist; BASF, Ludwigshafen, Deutschland) gemischt. Die beiden Vaccinen wurden an je 10-20 g schweren Mäusen zu je 0,5 ml appliziert. 3 Wochen nach der Vaccination (p.v.) wurden die Tiere entblutet und das Serum auf Antikörper gegen die einzelnen Antigene untersucht. Die Resultate der gefundenen Antikörpertiter sind nachfolgend tabellarisch aufgeführt.

| Antigen | ohne Adjuvans | Pluronic® L 61 |
|---|---|---|
| IBR | 0 | 1: 800 |
| Reo 1 | 0 | 1: 80 |
| PI 3 | 0 | 1:1280 |

Beispiel 2

10 ml inaktiviertes IBR Antigen wurden in 2 x 5 ml aufgeteilt und mit $AL(OH)_3$ oder Pluronic® L 101 (Verbindung der Formel I, wobei das Molekulargewicht dieser Verbindung 3.800 und das des Mittelteils 3.250 ist; BASF, Ludwigshafen, Deutschland) adjuviert.

Jede Vaccine wurde an je 10-20 g schweren Mäusen zu je 0,5 ml appliziert. 3 Wochen p.v. wurden die Tiere entblutet und die IBR-Antikörper im Serum bestimmt.

Die Antikörpertiter dieses Versuches sind tabellarisch aufgeführt.

| Antigen | $Al(OH)_3$ (Stand der Technik) | Pluronic® L 101 |
|---|---|---|
| IBR | 1:450 | 1:650 |

Aus diesen Beispielen geht die immunstimulatorische Wirkung der verwendeten Polyole hervor.

In den folgenden Beispielen wurde das Aujeszkyvirusmodell verwendet, ein in vivo Modell, bei dem die Schutzwirkung einer Vaccine gegen eine Belastungsinfektion der Maus bestimmt werden kann.

Beispiel 3

Inaktiviertes Aujeszkyvirusantigen wurde mit den in der folgenden Tabelle angegebenen Adjuvantien zu Vaccinen zusammengesetzt. Jede Vaccine wurde an je 5 Mäusen in Mengen von 0,4 ml, 0,2 ml, 0,1 ml und 0,05 ml appliziert. 3 Wochen p.v. wurden die Tiere mit virulentem Aujeszkyvirus belastet und die Anzahl der überlebenden Tiere als $PD_{50}$ (protective Dosis 50%) festgestellt. Die Resultate beinhaltet die folgende Tabelle.

| Adjuvans | $Al(OH)_3$ + Saponin (Stand der Technik) | Pluronic® L 101 |
|---|---|---|
| $PD_{50}$ | 1,38 | 3,56 |

Beispiel 4

Es wurde wie in Beispiel 3 vorgegangen. Die Anzahl der Adjuvantien wurde erweitert.

| Adjuvans | $PD_{50}$ | |
|---|---|---|
| $Al(OH)_3$ | 0 ) | |
| $Al(OH)_3$ + Saponin | 1,38) | Stand der Technik |
| $AlPO_4$ | 2,0 ) | |
| Pluronic® L 101 | 3,56 | |
| Pluronic® L 101 + $AlPO_4$ | 4,50 | |
| Span® 80 (Sorbitan monooleate) | 2,0 | |
| Span® 80 + $AlPO_4$ | 2,88 | |

Es zeigte sich überraschenderweise, daß sowohl die Pluronics® als auch das Monooleat Span® 80 eine bessere adjuvierende Wirkung als $Al(OH)_3$ aufweisen. Ferner zeigte sich, daß durch die Kombination von Pluronic® oder Monooleat mit bekannten Adjuvantien wie $AlPO_4$ ein deutlicher Summationseffekt der immunstimulatorischen Wirkung auftritt.

Patentansprüche

1. Verfahren zur Adjuvierung von ölfreien Vaccinen, dadurch gekennzeichnet, daß der Vaccine entweder eine Verbindung der Formel I

$$HO(CH_2CH_2O)_a(\underset{CH_3}{\underset{|}{C}}HCH_2O)_b(CH_2CH_2O)_cH \qquad (I)$$

mit einem Molekulargewicht zwischen 1.000 und etwa 15.000, wobei a und b statistisch gleich sind und das Verhältnis a zu b von 1:10 bis zu 1:16 reicht, oder ein Fettsäureester eines mehrwertigen Alkohols zugesetzt wird.

2. Verfahren nach Anspruch 1, dadurch gekennzeichnet, daß eine Verbindung der Formel I mit einem durchschnittlichen Molekulargewicht von 3.800, wobei das Molekulargewicht des Polypropylenmittelteils etwa 3.250 beträgt, zugesetzt wird.

3. Verfahren nach Anspruch 1, dadurch gekennzeichnet, daß eine Verbindung der Formel I mit einem durchschnittlichen Molekulargewicht von 2.000, wobei das Molekulargewicht des Mittelteils 1.750 beträgt, zugesetzt wird.

4. Verfahren nach Anspruch 1, dadurch gekennzeichnet, daß ein Ester einer Fettsäure mit 10 bis 20 Kohlenstoffatomen mit einem mehrwertigen Alkohol mit 3 bis 6 Kohlenstoffatomen zugesetzt wird.

5. Verfahren nach Anspruch 1, dadurch gekennzeichnet, daß ein Ester der Ölsäure, Stearinsäure, Palmitinsäure oder Laurinsäure mit Sorbitan, Mannitan, Hydroxygruppen tragenden Polyoxyalkylenen oder Glycerin zugesetzt wird.

6. Verfahren nach Anspruch 5, dadurch gekennzeichnet, daß der Ester ein Monoester ist.

7. Verfahren nach einem der Ansprüche 1 bis 6, dadurch gekennzeichnet, daß zusätzlich ein mineralisches Adjuvans zugesetzt wird.